# FASCICULE DE BREVET EUROPEEN

(11) **EP 0 727 228 B1**
(45) Date de publication et mention de la délivrance du brevet: **24.04.2002**
(21) Numéro de dépôt: 96420043.0
(22) Date de dépôt: 09.02.1996
(51) Int. Cl.: A61L 2/04, G01N 35/10

(54) **Dispositif de prélèvement et/ou éjection d'un milieu contaminant, avec décontamination de l'organe de manipulation dudit milieu**
Vorrichtung zur Entnahme und/oder Ausstoss eines verunreinigten Mediums mit Dekontaminierung der Behandlungsvorrichtung dieses Mediums
Collection and ejection device of contaminated medium with decontamination of handling device of said medium

(30) Priorité: 16.02.1995 FR 9502010
(43) Date de publication de la demande: 21.08.1996
(73) Titulaire: BIO MERIEUX, 69280 Marcy l'Etoile (FR)
(72) Inventeur: Colin, Bruno, 69280 Marcy l'Etoile (FR); Bossy, Geneviève, 01500 Amberieu (FR)
(74) Mandataire: Guerre, Dominique

(56) Documents cités:
- FR-A- 2 618 336

## Description

La présente invention concerne le prélèvement et/ou l'éjection d'un milieu contaminant, avec une décontamination de l'organe de manipulation dudit milieu, avant ou après chaque prélèvement et/ou éjection de ce dernier.

Par "milieu contaminant", on entend tout milieu appelé à être traité ou manipulé dans tout procédé, processus, ou méthode, par exemple d'analyse, mais devant être substantiellement éliminé ou supprimé, car indésirable à un moment ou à un autre dudit processus, procédé ou méthode, et ceci quelle que soit la forme physique ou présentation dudit milieu, par exemple qu'il s'agisse d'un matériau, matière, ou échantillon sous forme liquide, ou gazeuse, ou diphasique (liquide + gaz), ou encore sous forme de poudre, dès lors que ledit milieu peut être manipulé comme un fluide, notamment par aspiration et/ou refoulement. En conséquence de la définition précédente, "contaminer" et "décontaminer" signifient respectivement, mettre au contact ou apporter un milieu contaminant dans ou sur un objet, et éliminer ou supprimer le même milieu contaminant dudit objet. Et par "inerte", on entendra tout milieu, matériau ou matière exempt de tout milieu contaminant.

Au premier rang des milieux contaminants considérés par la présente invention, figurent les milieux biologiques, tels qu'échantillons de fluide ou prélèvement corporel, susceptibles de contenir ou d'être souillés par différents germes ou agents pathogènes, tels que virus, bactéries, ou autres cellules. Dans ce cas, et pour la description ci-après, "milieu contaminant" signifiera milieu non stérile, et "décontaminer" signifiera stériliser.

Mais la présente invention ne saurait être limitée à la stérilisation, puisque bien d'autres milieux contaminants peuvent être considérés selon l'invention, par exemple :
- tout matériel ou substance nucléique, telle qu'ADN, manipulé dans toute technique de biologie moléculaire,
- ou encore tout milieu organique, qu'il est nécessaire à la fois de manipuler et d'éliminer, à un moment ou à un autre, de tout procédé, par exemple d'analyse chimique ou biochimique.

Cependant, la caractéristique commune de tous les milieux contaminants considérés par la présente invention est que ceux-ci peuvent être détruits en portant leur température à une valeur relativement élevée, par exemple excédant 150°C. En conséquence, tous les milieux contaminants considérés selon l'invention peuvent être incinérés ou calcinés, en perdant de ce fait leurs caractéristiques ou propriétés qui les rendaient contaminants.

Dans différents procédés ou appareils d'analyse automatique, on doit à la fois prélever et/ou éjecter un milieu contaminant, et décontaminer l'organe de manipulation du milieu, avant ou après chaque prélèvement et/ou éjection dudit milieu. Ainsi, dans tout appareil automatisé d'analyse ou détection bactériologique, en traitant en même temps une pluralité d'échantillons biologiques d'origines différentes, par exemple de fluide corporel, l'organe de manipulation prélève chaque échantillon à partir d'un réceptacle le contenant, par exemple un tube à essai, pour l'éjecter ensuite avec ou sans dilution, dans une cassette d'analyse contenant différents réactifs, eux-mêmes contenus dans différents puits de titration respectivement ; dans ce cas, entre deux opérations élémentaires de prélèvement/éjection de deux échantillons respectivement différents, il est nécessaire de disposer d'un organe de manipulation stérile.

La solution a priori la plus simple, pour satisfaire l'exigence de stérilité, requise par la manipulation de deux échantillons contaminants successifs et respectivement différents, consiste a priori à prévoir ou disposer d'un nouvel organe de manipulation, entre deux opérations élémentaires de prélèvement et/ou d'éjection, cet organe de manipulation comportant une aiguille creuse de circulation et/ou rétention du milieu contaminant manipulé. En d'autres termes, selon cette solution, l'organe de manipulation constitue un composant consommable, remplacé ou jeté, après ou avant chaque opération élémentaire de prélèvement et/ou éjection du milieu contaminant traité.

Cette solution passe par la réalisation ou la disposition d'organes de manipulation, notamment d'aiguilles creuses de circulation et/ou rétention, réalisées dans une matière plastique, pour limiter leur coût de revient, et par conséquent par la mise en oeuvre de pièces plastiques à faible rigidité ou résistance mécanique, et par conséquent de manipulation délicate dans un processus automatisé.

Mais surtout, une telle solution passant par le remplacement de l'organe de manipulation à chaque opération de prélèvement et/ou éjection d'un milieu contaminant, s'avère difficile, voire impossible à automatiser, compte tenu notamment de la faible rigidité intrinsèque des aiguilles creuses de circulation et/ou rétention du milieu contaminant, soulignée précédemment. Cette automatisation passe à tout le moins par une complexité structurelle ou fonctionnelle des moyens y concourant.

La présente invention a pour objet une solution rompant avec l'approche précitée, et permettant de manière simple une automatisation de la décontamination de l'organe de manipulation du dispositif de prélèvement et/ou éjection du milieu contaminant, que ce dispositif soit utilisé de manière autonome, ou qu'il appartienne plus généralement à un appareil de traitement, notamment d'analyse, du ou des échantillons de milieu contaminant.

La présente invention se caractérise par la coopération des moyens suivants :
- l'aiguille creuse de l'organe de manipulation du milieu contaminant consiste essentiellement en un tube métallique, s'étendant d'une extrémité libre, dite active, à une extrémité inactive, déterminant avec cette dernière une section susceptible d'être contaminée puis décontaminée plusieurs fois, c'est-à-dire de manière répétée,
- des moyens de branchement électrique sont associés à l'aiguille creuse, pour faire circuler dans cette dernière un courant électrique, ceci pratiquement de son extrémité active à son extrémité inactive, c'est-à-dire dans toute la section de ladite aiguille susceptible d'être contaminée, aussi bien à l'intérieur qu'à l'extérieur du tube métallique,
- et des moyens de contrôle, notamment de type électrique, sont prévus et agencés pour apporter dans l'aiguille creuse une quantité prédéterminée d'énergie électrique, dosée en fonction des caractéristiques électriques du tube métallique, entre un maximum suffisant pour une décontamination de l'aiguille, dans sa section précitée, aussi bien à l'intérieur qu'à l'extérieur du tube métallique, et un maximum pour limiter l'échauffement de cette même aiguille, et préserver son intégrité, notamment sa forme originelle.

Par conséquent, grâce aux moyens de la présente invention, l'aiguille creuse et l'organe de manipulation qui la comprend, deviennent un composant permanent du dispositif de prélèvement et/ou éjection du milieu contaminant, pouvant être utilisé "à répétition" en quelque sorte, ce qui n'exclut pas bien évidemment un changement périodique de ce même organe de manipulation, pour des raisons d'entretien ou d'usure par exemple.

Conformément aux documents techniques WO-A-92/14096, US-A-5 300 752, FR-A-2 618 336 et EP-A-0 136 392, on a déjà proposé de chauffer par effet ohmique une aiguille de seringue hypodermique, aux fins de sa destruction complète, notamment par fusion et/ou déformation définitive du tube métallique la constituant, dont l'intérieur et/ou l'extérieur avaient été mis préalablement au contact d'un milieu contaminant. De manière générale, selon ces documents, on apporte par tout moyen approprié, un courant électrique, circulant de l'extrémité libre à l'autre extrémité de ladite aiguille, dans la paroi métallique et selon toute la longueur du tube la constituant ; ce courant dissipe dans le tube précité une énergie thermique par effet ohmique, conduisant à la fusion ou destruction complète, notamment avec déformation définitive, de l'aiguille de seringue.

Au contraire, selon la présente invention, l'énergie électrique apportée dans l'aiguille de prélèvement et/ou éjection du milieu contaminant est strictement contrôlée ou dosée, en fonction des caractéristiques électriques du tube métallique constituant ladite aiguille, d'une part pour suffire à une décontamination de l'aiguille, tant extérieure qu'intérieure, et d'autre part pour limiter le chauffage et préserver l'intégrité de l'aiguille, dont sa forme originelle.

Selon l'invention, il faut observer que le courant électrique peut être apporté dans l'aiguille, aussi bien par effet inductif, qu'en incluant l'aiguille creuse dans un circuit électrique, agencé pour faire circuler le courant électrique, substantiellement de l'extrémité active à l'extrémité inactive du tube métallique constituant ladite aiguille, et déterminant entre elles la section susceptible d'être contaminée puis décontaminée, de manière répétée.

La présente invention est décrite maintenant par référence aux dessins annexés, dans lesquels:
- la Figure 1 représente, de manière séparée et indépendante, un dispositif de prélèvement et/ou éjection d'un milieu contaminant, appartenant à un appareil de traitement, notamment analyse d'au moins un échantillon d'un milieu contaminant, non représenté par ailleurs,
- la Figure 2 représente plus particulièrement l'organe de manipulation du milieu contaminant, tel que mis en oeuvre dans le dispositif selon Figure 1,
- la Figure 3 représente une vue en coupe de l'organe de manipulation représenté à la Figure 2,
- les Figures 4 et 5 représentent des formes alternatives de l'organe de manipulation représenté aux Figures 2 et 3,
- la Figure 6 représente une vue en coupe du four d'incinération, faisant partie du dispositif de prélèvement et/ou éjection présenté à la Figure 1.

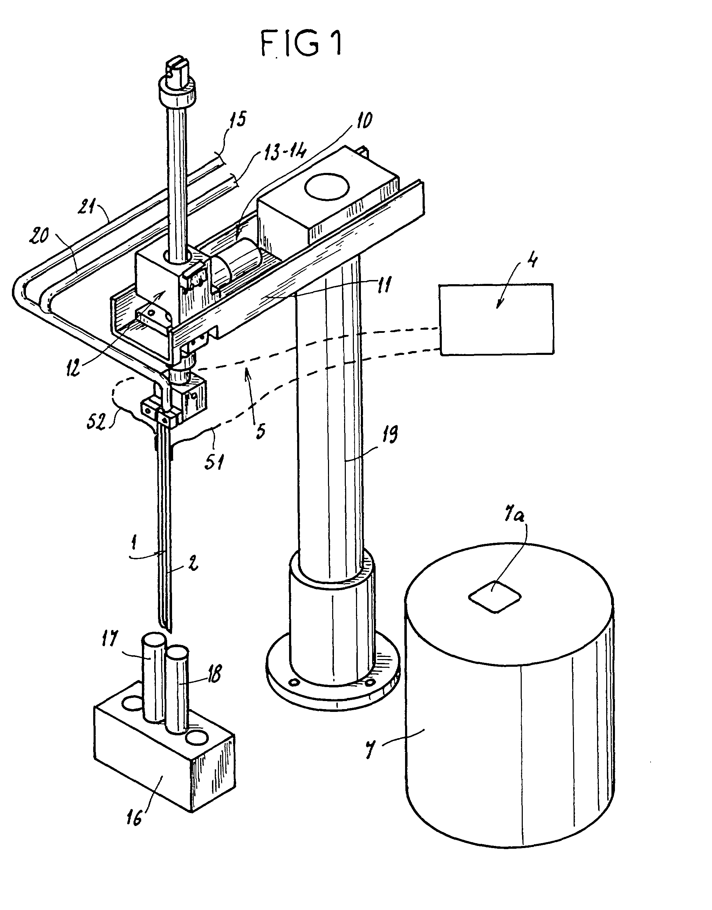

Conformément à la Figure 1, le dispositif selon l'invention permet de prélever un échantillon d'un milieu contaminant, contenu dans un tube 17, lui même supporté dans un porte-tube 16, puis d'éjecter l'échantillon prélevé dans un tube différent 18, dans lequel sera apporté également un diluant. Ce même dispositif permet de décontaminer l'organe de manipulation décrit ci-après, entre deux opérations de prélèvement et éjection de deux échantillons de milieux contaminants respectivement différents, présentés l'un à la suite de l'autre, en vis-à-vis de l'organe de manipulation précité, par exemple en présentant un nouveau support 16 contenant deux nouveaux tubes 17 et 18, dont le premier contient un nouvel échantillon différent, et le second demeure vide.

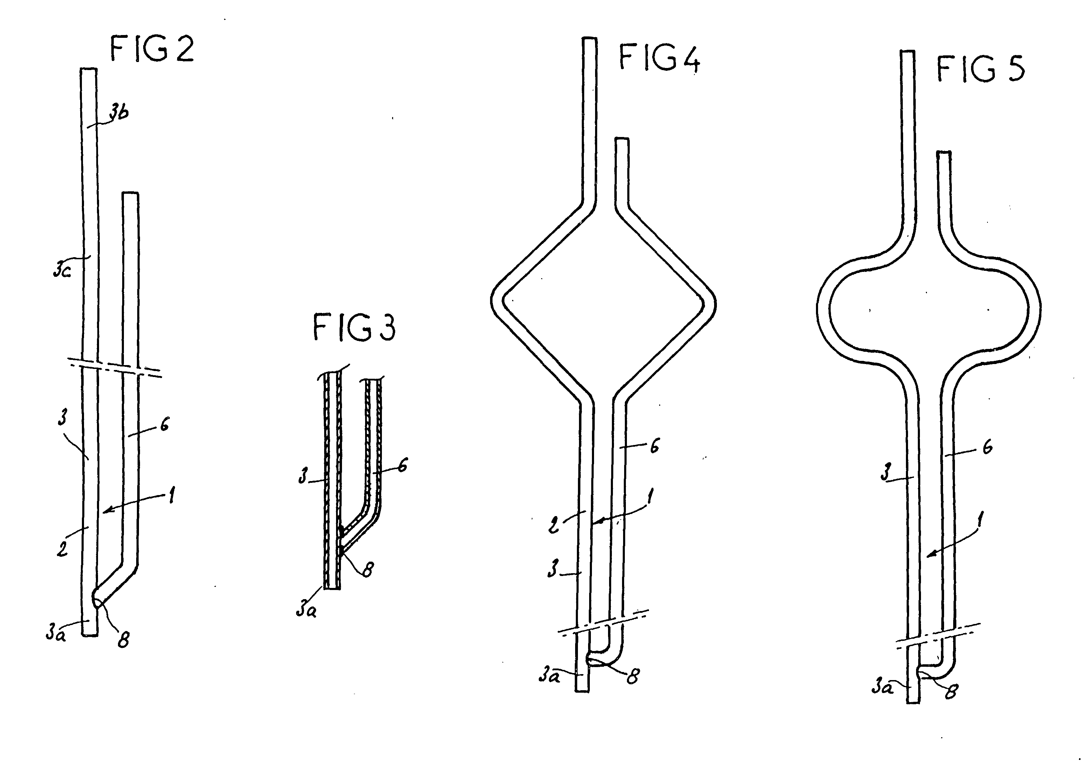

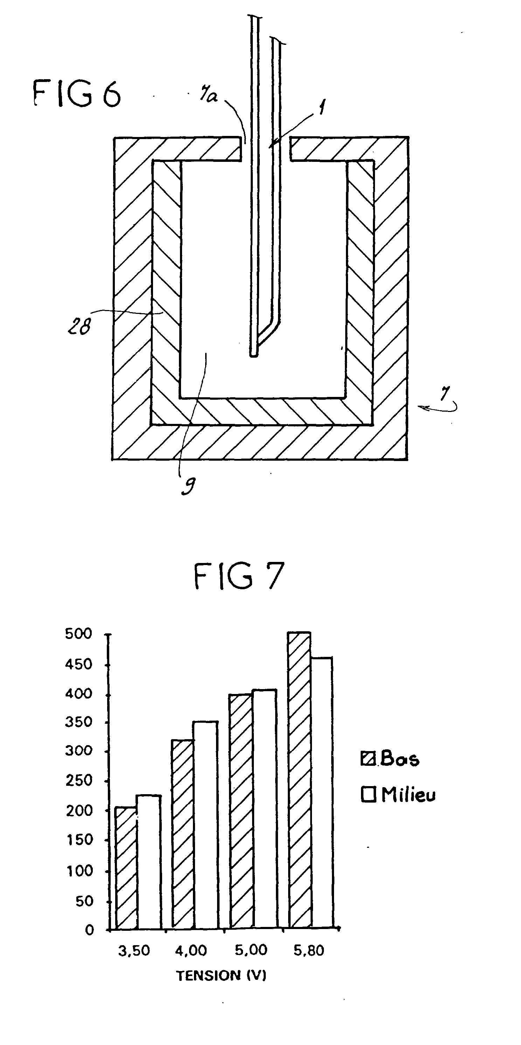

Le dispositif représenté de manière schématique et en perspective à la Figure 1 comprend de manière générale des organes ou composants suivants :
- un organe 1 de manipulation du milieu contaminant, plus particulièrement représenté aux Figures 2 et 3, comprenant une aiguille creuse 2 de circulation et/ou rétention du milieu contaminant, et une électrode 6,
- des moyens 5 de branchement électrique, associés à l'aiguille creuse 2 et à l'électrode 6, pour faire circuler un courant électrique dans ladite aiguille,
- un four 7 d'incinération, destiné à coopérer avec l'organe 1 de manipulation du milieu contaminant, agencé ou susceptible d'incinérer toute projection dudit milieu à partir de l'aiguille creuse 2,
- un moyen 10 de déplacement de l'organe 1 de manipulation du milieu contaminant, d'une position de prélèvement et/ou éjection de ce dernier, montrée par la Figure 1, à une position de décontamination, montrée par la Figure 6, dans laquelle l'organe 1 de manipulation est disposé à l'intérieur et au centre de l'enceinte 9 d'incinération, selon la Figure 6,
- des moyens de contrôle 4, prévus et agencés notamment pour contrôler la quantité d'énergie électrique apportée dans l'aiguille 2, mais aussi pour séquencer les différentes étapes ou opérations nécessaires à la mise en oeuvre du dispositif de prélèvement et/ou éjection, dont le passage de la position de prélèvement et/ou éjection, montrée à la Figure 1, à la position de décontamination, montrée à la Figure 6, et inversement.

L'aiguille creuse 2 consiste en un tube métallique 3, par exemple en inox ou tout autre matériau approprié, tel que des matériaux composites chargés, s'étendant d'une extrémité libre 3a, active au sens où c'est par cette extrémité que s'effectuent le prélèvement et/ou l'éjection du milieu contaminant, à une extrémité inactive 3b, déterminant avec cette dernière une section 3c, susceptible d'être contaminée puis décontaminée plusieurs fois, comme décrit ci-après.

L'organe de manipulation 1 comprend également une électrode 6, disposée parallèlement à l'aiguille creuse 2, et constituée par un tube métallique, ayant sensiblement les mêmes caractéristiques géométriques et électriques que le tube métallique 3 de l'aiguille creuse 2. Cette électrode 6 est en continuité électrique avec le tube métallique, dans une zone 8 de liaison avec cette dernière, adjacente à l'extrémité active 3a de l'aiguille 2 ; cette zone de liaison est obturée par un apport métallique, de manière à préserver l'étanchéité du tube 3 vis-à-vis de l'électrode creuse 6.

D'autres formes d'organes de manipulation sont montrées aux Figures 4 et 5 respectivement, en prévoyant à la fois dans le tube métallique 3 et l'électrode 6 une lyre de dilatation, la configuration du tube métallique 3 permettant par ailleurs d'éviter un reflux du milieu contaminant.

Les moyens 5 de branchement électrique comportent deux contacts électriques 51 et 52, respectivement avec l'extrémité inactive 3b de l'aiguille creuse 2, et avec l'extrémité de l'électrode 6, opposée à la zone de liaison 8, ce qui permet de faire circuler un courant électrique dans l'aiguille creuse 2, subtantiellement de son extrémité active 3a, ou de manière adjacente à cette dernière, à son extrémité inactive 3b, c'est-à-dire selon pratiquement toute la section 3c. De cette manière également, pratiquement toute l'électrode 6, située en termes de niveau dans la section 3c, se trouve également parcourue par le courant électrique de décontamination de l'aiguille creuse 2.

Comme représenté à la Figure 6, le four 7 d'incinération comprend une paroi chaude 28, mise en température par des moyens non représentés, tels qu'une résistance électrique, cette paroi déterminant une enceinte 9 d'incinération, autour et à distance de l'organe 1 de manipulation, lorsque ce dernier a pénétré dans le four 7 par son ouverture supérieure 7a. La température dans l'enceinte 9 d'incinération est contrôlée ou réglée pour permettre d'incinérer toute projection du milieu contaminant, à partir de l'extrémité ou de la face extérieure du tube métallique 3 de l'aiguille creuse 2.

Le moyen 10 de déplacement de l'organe 1 de manipulation du milieu contaminant comprend un bras 11 pivotant, monté à l'extrémité supérieure d'une potence 19, ce bras pouvant passer de la position de prélèvement et/ou éjection, montrée à la Figure 1, à la position de décontamination, montrée à la Figure 6, sous la commande du moyen de contrôle 4. A l'extrémité libre du bras 11 est monté et embarqué un organe 12 de déplacement vertical de l'organe 1 de manipulation, constitué de manière non représentée par une crémaillère entraînée par un moteur électrique. Dans la position de décontamination de la Figure 6, l'ouverture 7a du four 7 se trouve à l'aplomb de cet organe 12 de déplacement vertical.

Comme représenté de manière schématique à la Figure 1, l'extrémité 3b de l'aiguille creuse 2 de l'organe 1 de manipulation du milieu contaminant est connectée, par un même tuyau 20, d'une part à une source 13 d'aspiration et/ou refoulement du milieu contaminant, et d'autre part, à une source 14 d'un diluant du milieu contaminant, pouvant être éjecté par l'aiguille 2, et par un tuyau 21 à une source 15 d'un gaz inerte sous pression, pouvant être aspiré ou refoulé par l'aiguille 2.

Le moyen de contrôle 4 est agencé pour séquencer au moins deux opérations élémentaires, choisies parmi les opérations suivantes, en fonction du processus ou protocole de prélèvement et/ou éjection, avec décontamination, retenu par l'utilisateur :
- aspirer et/ou refouler, avec la source 13, le milieu contaminant par l'aiguille creuse 2,
- éjecter au moyen de la source 15, un flux de gaz inerte par l'aiguille creuse 2,
- refouler par l'aiguille creuse 2 un courant 14 du diluant,
- et passer de la position de prélèvement, et/ou la position d'éjection du milieu contaminant, à la position de décontamination, et inversement.

Ces mêmes moyens de contrôle permettent d'apporter dans l'aiguille creuse 2, mais aussi dans l'électrode 6, une quantité prédéterminée d'énergie électrique, dosée en fonction des caractéristiques électriques du tube 3 et de l'électrode 6, d'une part pour suffire à la décontamination de l'aiguille 2 et de l'électrode 6, tant intérieure qu'à extérieure, et d'autre par pour limiter l'échauffement et préserver l'intégrité, dont la forme d'origine de l'aiguille 2 et de l'électrode 6.

Grâce à la disposition de l'électrode 6, par rapport à l'aiguille creuse 3, plus particulièrement montrée à la Figure 3, pour décontaminer notamment l'aiguille creuse 2, on inclut donc cette dernière dans un circuit électrique, permettant de faire circuler un courant électrique, pratiquement de l'extrémité active 3a du tube 3, à l'extrémité inactive 3b de l'aiguille 2, séparée de l'extrémité 3a sur une distance déterminant la section 3c de l'aiguille 2 susceptible d'être contaminée puis décontaminée de manière répétée ; en effet, pendant la phase de décontamination, le courant électrique circule de la zone 8 de liaison, adjacente à l'extrémité active 3a, à l'extrémité inactive 3b de l'aiguille 2. Pendant la phase de chauffage de l'aiguille 2 pour la décontaminer, on dispose ladite aiguille dans l'enceinte 9 d'incinération, dont la paroi chaude 28 disposée autour et à distance de l'aiguille 2, permet d'incinérer toute projection de milieu contaminant, à partir de l'extrémité active 3a et/ou de la face extérieure du tube métallique 3 et/ou de la face extérieure de l'électrode 6.

Un dispositif de prélèvement et/ou éjection selon l'invention a été expérimenté en ce qui concerne ses performances de stérilisation.

L'aiguille creuse 2 est constituée par un tube métallique en inox, ayant une longueur utile par exemple d'environ 100 mm, de préférence 125 mm, une section externe comprise entre 0,5 et 3 mm, en particulier environ 2 mm. L'électrode 6 est constituée par le même tube métallique, ayant une longueur utile voisine de celle du tube métallique 3, par exemple d'environ 100 mm.

L'étape de stérilisation ou décontamination dans le four d'incinération, se compose des phases suivantes :
- soufflage d'air inerte par l'aiguille 2, sans chauffage ; chauffage par effet ohmique, avec soufflage d'air inerte ; et soufflage d'air inerte sans chauffage ; le tout en 6 secondes
- soufflage d'air inerte, pendant 3 secondes, pour refroidir l'aiguille 2 et l'électrode 6.

Le tableau selon Figure 7 donne les températures atteintes en °C, respectivement au bas et au milieu de l'aiguille creuse 2, en fonction de la tension électrique en V, appliquée à cette dernière.

Puis, en retenant la même durée de chauffage de l'aiguille creuse 2, comprise entre environ 1 et 5 secondes, et de préférence entre 2 et 3 secondes, pour une tension électrique appliquée à l'aiguille de 4V, on expérimente les performances de stérilisation du dispositif selon l'invention, par rapport à quatre microorganismes, à savoir *E. coli, P. aeruginosa, C. albicans, S. epidermidis*. Pour ce faire, le protocole suivant est utilisé :
- on contamine l'aiguille 2 avec un échantillon de 6.10⁸ cellules/ml de chaque microorganisme
- puis on stérilise l'aiguille 2 selon l'étape définie précédemment, et
- à la fin de l'étape de stérilisation, on fait passer un volume de 2,1 ml d'un diluant inerte (aseptique) par l'extrémité active 3a
- ce volume est divisé en fractions de 0,3 ml, ensemençant respectivement 7 boîtes comprenant un milieu nutritif agar-sang
- après une nuit d'incubation à une température comprise entre 30 et 42°C, de préférence 37°C, on identifie et décompte le microorganisme testé
- on dispose d'un contrôle positif (diluant avec le microorganisme considéré) et d'un témoin (le diluant seul)
- pour chaque microorganisme, on effectue une première série de 10 essais, et une deuxième série de 5 essais.

Dans ces conditions, après stérilisation, aucune contamination avec le microorganisme testé n'a été observée.

## Revendications

1. Procédé de chauffage d'une aiguille creuse (2) consistant essentiellement en un tube (3) métallique, dont l'intérieur et/ou l'extérieur ont été mis préalablement au contact d'un milieu contaminant, selon lequel on apporte un courant électrique dans ladite aiguille, circulant dans la paroi métallique et selon la longueur dudit tube (3), et dissipant dans ce dernier une énergie thermique par effet ohmique, **caractérisé en ce qu'**on apporte dans ladite aiguille (2) une quantité prédéterminée d'énergie électrique, dosée en fonction des caractéristiques électriques dudit tube (3), d'une part pour suffire à une décontamination de l'aiguille, tant intérieure qu'extérieure, et d'autre part pour limiter le chauffage et préserver l'intégrité, dont la forme originelle de ladite aiguille.

2. Procédé de décontamination par chauffage selon la revendication 1, **caractérisé en ce qu'**on apporte le courant électrique par effet inductif.

3. Procédé de décontamination par chauffage selon la revendication 1, **caractérisé en ce qu'**on inclut l'aiguille creuse (2) dans un circuit (5) électrique, agencé pour faire circuler un courant électrique, substantiellement de l'extrémité active (3a) du tube, à une extrémité inactive (3b) de ce dernier, distante de ladite extrémité active, et déterminant avec cette dernière une section susceptible d'être contaminée.

4. Procédé selon la revendication 3, **caractérisé en ce qu'**on fait circuler le courant électrique d'une zone (8) adjacente à l'extrémité active (3a), à ladite extrémité inactive (3b).

5. Procédé de décontamination par chauffage selon la revendication 1, **caractérisé en ce que**, pendant le chauffage de l'aiguille (2), on dispose cette dernière dans une enceinte (9) d'incinération, dont la paroi chaude (28), autour et à distance de ladite aiguille, est susceptible d'incinérer toute projection de milieu contaminant à partir de l'extrémité active (3a) et/ou de la face extérieure du tube métallique (4).

6. Dispositif de prélèvement et/ou éjection d'un milieu contaminant, comprenant un organe (1) de manipulation dudit milieu, comportant une aiguille creuse de circulation et/ou rétention dudit milieu, **caractérisé en ce que** l'aiguille creuse (2) consiste en un tube métallique (3) s'étendant d'une extrémité libre (3a) dite active à une extrémité inactive (3b), déterminant avec cette dernière une section (3c) susceptible d'être contaminée puis décontaminée plusieurs fois, des moyens (5) de branchement électrique sont associés à l'aiguille creuse (2), pour faire circuler un courant électrique dans cette dernière, substantiellement de son extrémité active (3a) à son extrémité inactive (3b), et des moyens de contrôle (4) sont prévus et agencés pour apporter dans ladite aiguille une quantité prédéterminée d'énergie électrique, dosée en fonction des caractéristiques électriques dudit tube (3), d'une part pour suffire à une décontamination de l'aiguille, tant intérieure qu'extérieure, et d'autre part pour limiter l'échauffement et préserver l'intégrité, dont la forme originelle de ladite aiguille.

7. Dispositif selon la revendication 5, **caractérisé en ce que** l'organe (1) de manipulation du milieu contaminant comprend une électrode (6) dont une extrémité est en continuité électrique avec le tube métallique (3), dans une zone (8) de liaison avec cette dernière, adjacente à son extrémité active (3a).

8. Dispositif selon la revendication 7, **caractérisé en ce que** l'électrode (6) est disposée parallèlement à l'aiguille creuse (2).

9. Disposition selon la revendication 7, **caractérisé en ce que** l'électrode (6) est un tube métallique, ayant sensiblement les mêmes caractéristiques géométriques et électriques que le tube métallique (3) de l'aiguille creuse (2).

10. Dispositif selon les revendications 6 et 7, **caractérisé en ce qu'**il comprend, d'une part, un four (7) d'incinération, comprenant une paroi chaude (28) déterminant une enceinte (9) d'incinération autour et à distance de l'organe (1) de manipulation du milieu contaminant, susceptible d'incinérer toute projection dudit milieu à partir de la face extérieure et/ou de l'extrémité 3a du tube métallique (3) de l'aiguille creuse (2), et/ou de la face extérieure de l'électrode 6, et d'autre part, un moyen (10) de déplacement de l'organe (1) de manipulation du milieu contaminant, d'une position de prélèvement et/ou éjection de ce dernier, à une position de décontamination, dans laquelle l'organe (1) de manipulation est disposé dans l'enceinte (9) d'incinération.

11. Dispositif selon la revendication 10, **caractérisé en ce que** le moyen (10) de déplacement de l'organe de manipulation du milieu contaminant comprend un bras (11) pivotant de la position de prélèvement et/ou éjection dudit milieu, à la position de décontamination.

12. Dispositif selon la revendication 11, **caractérisé en ce qu'**un organe (12) de déplacement vertical de l'organe (1) de manipulation du milieu contaminant est embarqué par le bras pivotant (11), et le four (7) comporte une ouverture (7a) à l'aplomb dudit organe de déplacement vertical, dans la position de décontamination.

13. Dispositif selon la revendication 6, **caractérisé en ce que** l'aiguille (2) creuse de l'organe (1) de manipulation du milieu contaminant est connectée, du côté de son extrémité inactive (3b) avec une source (13) d'aspiration et/ou refoulement du milieu contaminant.

14. Dispositif selon la revendication 6, **caractérisé en ce que** l'aiguille creuse de l'organe (1) de manipulation du milieu contaminant est connectée, du côté de son extrémité inactive avec une source (15) d'un gaz inerte sous pression.

15. Dispositif selon la revendication 6, **caractérisé en ce que** l'aiguille creuse de l'organe (1) de manipulation du milieu contaminant est connectée, du côté de son extrémité inactive avec une source (14) d'un diluant du milieu contaminant.

16. Dispositif selon les revendications 6, 10, 13, 14 et 15, **caractérisé en ce que** les moyens (4) de contrôle sont agencés pour séquencer au moins deux opérations, choisies parmi les opérations suivantes :
- aspirer et/ou refouler (13) le milieu contaminant, par l'aiguille (2) creuse de circulation et/ou rétention dudit milieu
- éjecter un flux de gaz inerte (15) par l'aiguille creuse
- refouler un courant (14) du diluant par l'aiguille creuse
- passer de la position de prélèvement et/ou éjection du milieu contaminant, à la position de décontamination, et inversement.

17. Appareil de traitement, notamment analyse, d'au moins un échantillon d'un milieu contaminant, comprenant un dispositif de prélèvement et/ou éjection dudit échantillon selon l'une quelconque des revendications 6 à 16.

18. Organe (1) de manipulation d'un milieu contaminant, comprenant une aiguille creuse (2) de circulation et/ou rétention dudit milieu, **caractérisé en ce que** l'aiguille creuse (2) consiste essentiellement en un tube métallique (3) s'étendant d'une extrémité libre (3a) dite active, à une extrémité inactive (3b), déterminant avec cette dernière une section (3c) susceptible d'être contaminée, puis décontaminée plusieurs fois et **en ce que** l'organe (1) de manipulation du milieu contaminant comprend une électrode (6) dont une extrémité est en continuité électrique avec le tube métallique (3), dans une zone (8) de liaison avec cette dernière, adjacente à son extrémité active (3a).

19. Organe selon la revendication 18, **caractérisé en ce que** l'électrode (6) est disposée parallèlement à l'aiguille creuse (2).

## Claims

1. Process for heating a hollow needle (2) consisting essentially of a metal tube (3), the inside and/or outside of which have previously been brought into contact with a contaminating medium, according to which an electric current is supplied to the said needle, flowing in the metal wall and over the length of the said tube (3) and dissipating thermal energy therein through the ohmic effect, **characterized in that** a predetermined quantity of electrical energy is supplied to the said needle (2), metered as a function of the electrical characteristics of the said tube (3), on the one hand to suffice for decontamination of the needle, both inside and outside, and on the other hand to limit the heating and preserve the integrity, including the original shape, of the said needle.

2. Process for decontamination by heating according to Claim 1, **characterized in that** the electric current is supplied through the inductive effect.

3. Process for decontamination by heating according to Claim 1, **characterized in that** the hollow needle (2) is enclosed within an electric circuit (5) designed to make an electric current flow substantially from the active end (3a) of the tube to an inactive end (3b) thereof, distant from the said active end, and determining with the latter a section able to be contaminated.

4. Process according to Claim 3, **characterized in that** the electric current is made to flow from a region (8) adjacent to the active end (3a) to the said inactive end (3b).

5. Process for decontamination by heating according to Claim 1, **characterized in that**, during the heating of the needle (2), the latter is arranged inside an incineration vessel (9), whose hot wall (28), around and at some distance from the said needle, is able to incinerate any squirt of contaminating medium from the active end (3a) and/or from the outside face of the metal tube (4).

6. Device for withdrawing and/or ejecting a contaminating medium, comprising a member (1) for manipulating the said medium, including a hollow needle for the flow and/or retention of the said medium, **characterized in that** the hollow needle (2) consists of a metal tube (3) extending from a free so-called active end (3a) to an inactive end (3b), determining with the latter a section (3c) able to be contaminated and then decontaminated several times, means (5) for electrical connection are associated with the hollow needle (2), in order to make an electric current flow therein, substantially from its active end (3a) to its inactive end (3b), and control means (4) are provided and designed to supply the said needle with a predetermined quantity of electrical energy, metered as a function of the electrical characteristics of the said tube (3), on the one hand to suffice for decontamination of the needle, both inside and outside, and on the other hand to limit heat-up and preserve the integrity, including the original shape, of the said needle.

7. Device according to Claim 5, **characterized in that** the member (1) for manipulating the contaminating medium comprises an electrode (6), one end of which is in electrical continuity with the metal tube (3), in a region (8) of linkage with this end, adjacent to its active end (3a).

8. Device according to Claim 7, **characterized in that** the electrode (6) is arranged parallel to the hollow needle (2).

9. Arrangement according to Claim 7, **characterized in that** the electrode (6) is a metal tube, having substantially the same geometrical and electrical characteristics as the metal tube (3) of the hollow needle (2).

10. Device according to Claims 6 and 7, **characterized in that** it comprises, on the one hand, an incinerator (7), comprising a hot wall (28) determining an incineration vessel (9) around and at some distance from the member (1) for manipulating the contaminating medium, able to incinerate any squirt of the said medium from the outside face and/or from the end 3a of the metal tube (3) of the hollow needle (2), and/or from the outside face of the electrode 6, and on the other hand, a means (10) for displacing the member (1) for manipulating the contaminating medium, from a position for withdrawing and/or ejecting the medium, to a decontaminating position in which the manipulating member (1) is arranged in the incineration vessel (9).

11. Device according to Claim 10, **characterized in that** the means (10) for displacing the member for manipulating the contaminating medium comprises an arm (11) pivoting from the position for withdrawing and/or ejecting the said medium, to the decontaminating position.

12. Device according to Claim 11, **characterized in that** a member (12) for vertical displacement of the member (1) for manipulating the contaminating medium is carried by the pivoting arm (11), and the incinerator (7) includes an opening (7a) plumb with the said member for vertical displacement, in the decontaminating position .

13. Device according to Claim 6, **characterized in that** the hollow needle (2) of the member (1) for manipulating the contaminating medium is connected, on the side of its inactive end (3b), to a source (13) for sucking up and/or expelling the contaminating medium.

14. Device according to Claim 6, **characterized in that** the hollow needle of the member (1) for manipulating the contaminating medium is connected, on the side of its inactive end, to a source (15) of a pressurized inert gas.

15. Device according to Claim 6, **characterized in that** the hollow needle of the member (1) for manipulating the contaminating medium is connected, on the side of its inactive end, to a source (14) of a diluent of the contaminating medium.

16. Device according to Claims 6, 10, 13, 14 and 15, **characterized in that** the control means (4) are designed to sequence at least two operations, chosen from the following operations:
- sucking up and/or expelling (13) the contaminating medium, through the hollow needle (2) for the flow and/or retention of the said medium
- ejecting a flux of inert gas (15) through the hollow needle
- expelling a stream (14) of the diluent through the hollow needle
- passing from the position of withdrawal and/or ejection of the contaminating medium to the decontaminating position , and vice versa.

17. Apparatus for treating, especially analysing, at least one sample of a contaminating medium, comprising a device for withdrawing and/or ejecting the said sample according to any one of Claims 6 to 16.

18. Member (1) for manipulating a contaminating medium, comprising a hollow needle (2) for the flow and/or retention of the said medium, **characterized in that** the hollow needle (2) consists essentially of a metal tube (3) extending from a free so-called active end (3a) to an inactive end (3b), determining with the latter a section (3c) able to be contaminated and then decontaminated several times and **in that** the member (1) for manipulating the contaminating medium comprises an electrode (6), one end of which is in electrical continuity with the metal tube (3), in a region (8) of linkage with this end, adjacent to its active end (3a).

19. Member according to Claim 18, **characterized in that** the electrode (6) is arranged parallel to the hollow needle (2).

## Patentansprüche

1. Verfahren bei dem eine Hohlnadel (2) erwärmt wird, die im wesentlichen aus einem Metallrohr (3) besteht, dessen Inneres und/oder Äußeres zuvor in Kontakt mit einem kontaminierenden Medium gebracht wurde, gemäß dem an der Nadel ein elektrischer Strom angelegt wird, der in der Metallwand und der längs des Rohres (3) fließt, und der in dem Rohr durch Ohm'schen Effekt thermische Energie abgibt, **dadurch gekennzeichnet, daß** an die Nadel (2) eine bestimmte Menge elektrischer Energie angelegt wird, die in Abhängigkeit der elektrischen Eigenschaften des Rohres (3) so dosiert wird, daß diese einerseits für eine Dekontamination der Nadel sowohl innen als auch außen ausreicht, und daß andererseits die Erwärmung begrenzt wird und daß die Unversehrtheit der Nadel, darunter deren ursprüngliche Form, erhalten bleibt.

2. Verfahren zur Dekontamination durch Erwärmung nach Anspruch 1, **dadurch gekennzeichnet, daß** der elektrische Strom durch induktiven Effekt erzeugt wird.

3. Verfahren zur Dekontamination durch Erwärmung nach Anspruch 1, **dadurch gekennzeichnet, daß** die Hohlnadel (2) in einen Stromkreis (5) eingeschlossen wird, der so gestaltet ist, daß ein elektrischer Strom im wesentlichen von dem aktiven Ende (3a) des Rohres zu einem von dem aktiven Ende beabstandeten inaktiven Ende (3b) des Rohrs fließt, wobei das inaktive Ende mit dem aktiven Ende einen Abschnitt festlegt, der kontaminiert sein kann.

4. Verfahren nach Anspruch 3, **dadurch gekennzeichnet, daß** man den elektrischen Strom von einem Bereich (8), der an das aktive Ende (3a) angrenzt, zu dem inaktiven Ende (3b) fließen läßt.

5. Verfahren zur Dekontamination durch Erwärmung nach Anspruch 1, **dadurch gekennzeichnet, daß** die Nadel (2) während ihrer Erwärmung in einen Veraschungsbehältnis (9) eingebracht wird, dessen warme Wand (28), die um die Nadel herum und zu dieser beabstandet angeordnet ist, in der Lage ist, sämtlichen Auswurf des kontaminierenden Mediums, ausgehend von dem aktiven Ende (3a) und/oder von der Außenseite des Metallrohrs (4), zu veraschen.

6. Vorrichtung zur Entnahme und/oder zum Ausstoß eines kontaminierenden Mediums, die ein Element (1) zur Handhabung des Mediums aufweist, das eine Hohlnadel zur Zirkulation und/oder Retention des Mediums beinhaltet, **dadurch gekennzeichnet, daß** die Hohlnadel (2) aus einem Metallrohr (3) besteht, das sich von einem freien, sogenannten aktiven Ende (3a) zu einem inaktiven Ende (3b) erstreckt, wobei das inaktive Ende mit dem aktiven Ende einen Abschnitt (3c) festlegt, der kontaminiert sein kann und anschließend mehrfach dekontaminiert werden kann, wobei Stromversorgungsmittel (5) mit der Hohlnadel (2) verbunden sind, damit in dieser ein elektrischer Strom im wesentlichen von ihrem aktiven Ende (3a) zu ihrem inaktiven Ende (3b) fließt, und wobei Steuermittel (4) vorgesehen und angeordnet sind, um in die Nadel eine festgelegte Menge elektrischer Energie einzubringen, die in Abhängigkeit der elektrischen Eigenschaften des Rohres (3) dosiert wird, so daß diese einerseits für eine Dekontamination der Nadel sowohl innerlich als auch äußerlich ausreicht, und daß andererseits die Erwärmung begrenzt wird und die Unversehrtheit der Nadel, darunter deren ursprüngliche Form, erhalten bleibt.

7. Vorrichtung nach Anspruch 6, **dadurch gekennzeichnet, daß** das Element (1) zur Handhabung des kontaminierenden Mediums eine Elektrode (6) aufweist, deren eines Ende mit dem Metallrohr (3) in einem Verbindungsbereich (8), die an dessen aktives Ende (3a) angrenzt, in elektrisch leitender Verbindung steht.

8. Vorrichtung nach Anspruch 7, **dadurch gekennzeichnet, daß** die Elektrode (6) parallel zu der Hohlnadel (2) angeordnet ist.

9. Vorrichtung nach Anspruch 7, **dadurch gekennzeichnet, daß** die Elektrode (6) ein Metallrohr ist, das etwa die gleichen geometrischen und elektrischen Eigenschaften wie das Metallrohr (3) der Hohlnadel (2) aufweist.

10. Vorrichtung nach Anspruch 6 oder 7, **dadurch gekennzeichnet, daß** sie zum einen einen Veraschungsofen (7) mit einer warmen Wand (28) aufweist, die einen Veraschungsraum (9) um das Element (1) zur Handhabung des kontaminierenden Mediums herum und zu diesem beabstandet festlegt, und die in der Lage ist, sämtlichen Auswurf des Mediums, ausgehend von der Außenseite und/oder dem Ende (3a) des Metallrohrs (3) der Hohlnadel (2) und/oder von der Außenseite der Elektrode (6) zu veraschen, und daß sie zum anderen ein Bewegungsmittel (10) aufweist, mit dem das Element (1) zur Handhabung des kontaminierenden Mediums von einer Position zur Entnahme und/oder zum Ausstoß des Mediums in eine Position zur Dekontamination, in der das Element (1) zur Handhabung in den Veraschungsraum (9) eingebracht wird, bewegt werden kann.

11. Vorrichtung nach Anspruch 10, **dadurch gekennzeichnet, daß** das Bewegungsmittel (10) für das Element zur Handhabung des kontaminierenden Mediums einen Arm (11) aufweist, der von der Position zur Entnahme und/oder zum Ausstoß des Mediums in die Position zur Dekontamination schwenkbar ist.

12. Vorrichtung nach Anspruch 11, **dadurch gekennzeichnet, daß** ein Element (12) zur Höhenverstellung des Elements (1) zur Handhabung des kontaminierenden Mediums in den schwenkbaren Arm (11) eingebracht ist, und der Ofen (7) eine Öffnung (7a) beinhaltet, die in der Position zur Dekontamination im Lot des Elements zur Höhenverstellung ist.

13. Vorrichtung nach Anspruch 6, **dadurch gekennzeichnet, daß** die Hohlnadel (2) des Elements (1) zur Handhabung des kontaminierenden Mediums in der Nähe ihres inaktiven Endes (3b) mit einer Quelle (13) zum Ansaugen und/oder Auswerfen des kontaminierenden Mediums verbunden ist.

14. Vorrichtung nach Anspruch 6, **dadurch gekennzeichnet, daß** die Hohlnadel des Elements (1) zur Handhabung des kontaminierenden Mediums in der Nähe ihres inaktiven Endes mit einer Quelle (15) eines unter Druck stehenden inerten Gases verbunden ist.

15. Vorrichtung nach Anspruch 6, **dadurch gekennzeichnet, daß** die Hohlnadel des Elements (1) zur Handhabung des kontaminierenden Mediums in der Nähe ihres inaktiven Endes mit einer Quelle (14) eines Verdünnungsmittels für das kontaminierende Medium verbunden ist.

16. Vorrichtung nach einem der Ansprüche 6, 10, 13, 14 oder 15, **dadurch gekennzeichnet, daß** die Steuermittel (4) so angeordnet sind, daß zumindest zwei Arbeitsgänge, ausgewählt aus den folgenden Arbeitsgängen, ausgeführt werden:
- Ansaugen und/oder Auswerfen (13) des kontaminierenden Mediums durch die Hohlnadel (2) zur Zirkulation und/oder Retention des Mediums;
- Ausstoßen eines Stroms von inertem Gas (15) durch die Hohlnadel;
- Durchdrücken eines Stroms (14) eines Lösungsmittels durch die Hohlnadel;
- Übergang von der Position zur Entnahme und/oder zum Ausstoß des kontaminierenden Mediums in die Position zur Dekontamination, und umgekehrt.

17. Gerät zur Behandlung, insbesondere zur Analyse, von zumindest einer Probe eines kontaminierenden Mediums, das eine Vorrichtung zur Entnahme und/oder zum Ausstoß der Probe nach einem der Ansprüche 6 bis 16 aufweist.

18. Element (1) zur Handhabung eines kontaminierenden Mediums, das eine Hohlnadel (2) zur Zirkulation und/oder Retention des Mediums aufweist, **dadurch gekennzeichnet, daß** die Hohlnadel (2) im wesentlichen aus einem Metallrohr (3) besteht, das sich von einem freien, sogenannten aktiven Ende (3a) zu einem inaktiven Ende (3b) erstreckt, wobei das inaktive Ende mit dem aktiven Ende einen Abschnitt (3c) festlegt, der kontaminiert sein kann und anschließend mehrfach dekontaminiert werden kann, und daß das Element (1) zur Handhabung des kontaminierenden Mediums eine Elektrode (6) aufweist, deren eines Ende mit dem Metallrohr (3) in einer Verbindungszone (8), die an dessen aktives Ende (3a) angrenzt, in elektrisch leitender Verbindung steht.

19. Element nach Anspruch 18, **dadurch gekennzeichnet, daß** die Elektrode (6) parallel zu der Hohlnadel (2) angeordnet ist.
